# EUROPÄISCHE PATENTSCHRIFT

(11) **EP 0 602 573 B1**
(45) Veröffentlichungstag und Bekanntmachung des Hinweises auf die Patenterteilung: **12.06.1996**
(21) Anmeldenummer: 93120029.9
(22) Anmeldetag: 11.12.1993
(51) Int. Cl.: C07C 43/303, C07C 41/56

(54) **Verfahren zur Herstellung von Acetalen**
Process for the preparation of acetals
Procédé pour la préparation d'acétals

(30) Priorität: 17.12.1992 DE 4242677
(43) Veröffentlichungstag der Anmeldung: 22.06.1994
(73) Patentinhaber: BASF Aktiengesellschaft, D-67063 Ludwigshafen (DE)
(72) Erfinder: Hetterich, Manfred, D-67105 Schifferstadt (DE); Scholz, Hans-Ulrich, Dr., D-67273 Weisenheim (DE); Fuchs, Hartwig, D-67063 Ludwigshafen (DE); Lauterbach, Gerald, Dr., D-64625 Bensheim (DE)

(56) Entgegenhaltungen:
- EP-A- 0 144 815
- DE-A- 2 512 553
- DE-A- 2 625 074
- US-A- 3 969 385

## Beschreibung

Die vorliegende Erfindung betrifft ein verbessertes Verfahren zur Herstellung von Acetalen aus Aldehyden, die mindestens 8 Kohlenstoffatome tragen und die olefinisch ungesättigt sind oder durch Wasserabspaltung in olefinisch ungesättigte Aldehyde übergehen, durch Umsetzung mit Alkoholen in Gegenwart von Mineralsäuren als Katalysatoren.

Es ist allgemein bekannt (z.B. Houben-Weyl, Thieme Verlag, 1965, Bd. VI/3, S. 199), daß Acetale aus Aldehyden und Alkoholen in Gegenwart von Säuren hergestellt werden können.

Aus der DE 2625074 A1 ist ein solches Verfahren bekannt, bei dem spezielle ungesättigte Alkohole eingesetzt werden.

Für empfindliche Aldehyde allerdings ist diese allgemeine Methode nicht ohne Probleme anwendbar. So kommt es bei Aldehyden, die olefinisch ungesättigt sind oder durch Wasserabspaltung in olefinisch ungesättigte Aldehyde übergehen, zu unerwünschten Nebenreaktionen, die die Ausbeute vermindern und insbesondere bei Duftstoffen die Produktqualität negativ beeinflussen.

Aus der US 3969385 sind spezielle Ester als Insektizide bekannt.

Es bestand daher die Aufgabe, ein Verfahren bereitzustellen, das diese Nachteile vermeidet.

Demgemäß wurde eine Verbesserung des Verfahrens zur Herstellung von Acetalen aus Aldehyden, die mindestens 8 Kohlenstoffatome tragen und die olefinisch ungesättigt sind oder durch Wasserabspaltung in olefinisch ungesättigte Aldehyde übergehen, durch Umsetzung mit Alkoholen in Gegenwart von Mineralsäuren als Katalysatoren gefunden, die dadurch gekennzeichnet ist, daß die Säurekonzentration im Reaktionsgemisch bei 5 bis 200 ppm gehalten wird und als Alkohole C₁-C₄-Alkylalkohole eingesetzt werden.

Als Ausgangsstoffe im vorliegenden Verfahren dienen Aldehyde mit mehr als 8 Kohlenstoffatomen, die olefinisch ungesättigt sind, wie Dimethyloctenale, vorzugsweise Citronellal (3,7-Dimethyl-6-octenal) und Citral (3,7-Dimethyl-2,6-octadienal), sowie weiterhin solche Aldehyde, die durch Wasserabspaltung in olefinisch ungesättigte Aldehyde übergehen, wie Dimethylhydroxyoctenale, vorzugsweise 7-Hydroxycitronellal (3,7-Dimethyl-7-hydroxyoctanal).

Diese Aldehyde werden mit Alkoholen umgesetzt. Es handelt sich dabei um C₁-C₄-Alkylalkohole wie Ethanol, n-Propanol, n-Butanol und besonders bevorzugt um Methanol.

Als Mineralsäuren kommen z.B. Salzsäure, Salpetersäure, Bromwasserstoffsäure, Phosphorsäure, bevorzugt aber Schwefelsäure in Betracht. Bei der Herstellung von Acetalen mit einwertigen Alkoholen werden mindestens 2 Äquivalente Alkohole pro Äquivalent Aldehyd benötigt. In der Regel wird aber ein Überschuß an Alkohol verwendet, um die Reaktionsgeschwindigkeit zu steigern. Es können 2 bis 20, vorzugsweise 5 bis 10 Äquivalente Alkohol pro Äquivalent Aldehyd eingesetzt werden. Größere Mengen stören nicht, sind aber nicht notwendig.

Die Säuremenge beträgt 5 bis 200 ppm, vorzugsweise 10 bis 50 ppm, bezogen auf den Reaktionsansatz. Bei wesentlich höheren Säurekonzentrationen tritt vermehrt Nebenproduktbildung ein, bei niedrigeren Säurekonzentrationen ist die Reaktionsgeschwindigkeit unbefriedigend.

Im allgemeinen werden Aldehyd und Alkohol vermischt und dann mit der Säure, gegebenenfalls in einem Alkohol gelöst, versetzt.

Die Reaktion kann in inerten Lösungsmitteln wie Toluol und Cyclohexan durchgeführt werden. Bevorzugt ist es aber, sie in einem Überschuß des umzusetzenden Alkohols vorzunehmen.

Die Reaktion wird in der Regel bei der Siedetemperatur des eingesetzten Alkohols durchgeführt. Zur Erzielung von angemessenen Reaktionszeiten genügen aber in den meisten Fällen Temperaturen von 50 bis 70°C.

Da der Druck keinen merklichen Einfluß auf die Reaktion hat, wird diese normalerweise bei Normaldruck durchgeführt.

Die Reaktionszeit beträgt in den meisten Fällen 0,2 bis 4 h.

Die Reaktion kann kontinuierlich oder diskontinuierlich durchgeführt werden.

Die Aufarbeitung der Reaktionsmischung geschieht nach bekannten Methoden, vorzugsweise destillativ. Es hat sich bewährt, bei einer destillativen Aufarbeitung die Säure zuvor durch Zugabe von Basen wie Natriummethanolat zu neutralisieren.

Das erfindungsgemäße Verfahren erlaubt die Herstellung von Acetalen aus empfindlichen Aldehyden, wobei die Nebenproduktbildung weitgehend unterdrückt wird.

Von besonderer Bedeutung ist das Verfahren für solche Acetale, die als Duftstoffe dienen, wie Citronellaldimethylacetal und 7-Hydroxycitronellaldimethylacetal.

### Beispiele

### Beispiel 1

### Herstellung von 7-Hydroxycitronellaldimethylacetal

260 g Methanol und 150 g 7-Hydroxycitronellal (molares Verhältnis 9,3 : 1) wurden mit Schwefelsäure versetzt und auf 70°C erhitzt. Nach Ende der Reaktion wurde die Säure mit Natriummethanolatlösung neutralisiert und destilliert. Weitere Einzelheiten können Tabelle 1 entnommen werden.

**Tabelle 1**

| | Erfindungsgemäß | Vergleich |
|---|---|---|
| Säurekonzentration [ppm] | 10 | 1000 |
| Reaktionszeit [h] | 3 | 3 |
| Umsatz [%] | 87 | 94 |
| Nebenprodukte [%] | 0 | 3,3 |
| Selektivität [%] | 100 | 96 |

### Beispiel 2

### Herstellung von Citronellaldimethylacetal

In Analogie zu Beispiel 1 wurden 160 g Methanol und 80 g Citronellal mit Schwefelsäure versetzt und auf 68°C erhitzt. Nach Ende der Reaktion wurde die Säure mit Natriummethylatlösung neutralisiert und destilliert. Weitere Einzelheiten können Tabelle 2 entnommen werden.

**Tabelle 2**

| | Erfindungsgemäß | Vergleich |
|---|---|---|
| Säurekonzentration [ppm] | 10 | 1000 |
| Reaktionszeit [h] | 0,3 | 0,3 |
| Umsatz [%] | 93 | 98 |
| Nebenprodukte [%] | 6 | 33 |
| Selektivität [%] | 94 | 66 |

## Patentansprüche

1. Verfahren zur Herstellung von Acetalen aus Aldehyden, die mindestens 8 Kohlenstoffatome tragen und die olefinisch ungesättigt sind oder durch Wasserabspaltung in olefinisch ungesättigte Aldehyde übergehen, durch Umsetzung mit Alkoholen in Gegenwart von Mineralsäuren als Katalysator, dadurch gekennzeichnet, daß die Säurekonzentration im Reaktionsgemisch bei 5 bis 200 ppm gehalten wird und als Alkohole C₁-C₄-Alkylalkohole eingesetzt werden.

2. Verfahren nach Anspruch 1, dadurch gekennzeichnet, daß man als Aldehyd Citronellal, 7-Hydroxycitronellal oder Citral verwendet.

3. Verfahren nach Anspruch 1 oder 2, dadurch gekennzeichnet, daß man als Alkohol Methanol verwendet.

4. Verfahren nach den Ansprüchen 1 bis 3, dadurch gekennzeichnet, daß man als Mineralsäure Schwefelsäure verwendet.

## Claims

1. Process for the preparation of acetals from aldehydes which carry at least 8 carbon atoms and which are olefinically unsaturated or are converted into olefinically unsaturated aldehydes as a result of the elimination of water, by reaction with alcohols in the presence of mineral acids as catalyst, which comprises keeping the acid concentration in the reaction mixture at from 5 to 200 ppm and using C₁-C₄-alkyl alcohols as the alcohols.

2. Process as claimed in claim 1, wherein the aldehyde used is cintronellal, 7-hydroxycitronellal or citral.

3. Process as claimed in claim 1 or 2, wherein the alcohol used is methanol.

4. Process as claimed in any of claims 1 to 3, wherein the mineral acid used is sulfuric acid.

## Revendications

1. Procédé pour la préparation d'acétals à partir d'aldéhydes, qui portent au moins 8 atomes de carbone et ont des insaturations oléfiniques ou se transforment par élimination d'eau en des aldéhydes à insaturations oléfiniques, par réaction avec des alcools en présence d'acides minéraux comme catalyseur, caractérisé par le fait que la concentration en acide dans le mélange réactionnel est maintenue entre 5 et 200 ppm et qu'on emploie comme alcools des alkyl(C1-C4)alcools.

2. Procédé selon la revendication 1, caractérisé par le fait qu'on utilise, comme aldéhyde, du citronellal, du 7-hydroxycitronellal ou du citral.

3. Procédé selon l'une des revendications 1 ou 2, caractérisé par le fait qu'on utilise, comme alcool, du méthanol.

4. Procédé selon l'une quelconque des revendications 1 à 3, caractérisé par le fait qu'on utilise, comme acide minéral, de l'acide sulfurique.
